# EUROPEAN PATENT SPECIFICATION

(11) **EP 1 505 973 B1**
(45) Date of publication and mention of the grant of the patent: **03.03.2010**
(21) Application number: 03728969.1
(22) Date of filing: 16.05.2003
(51) Int. Cl.: A61K 31/454, A61K 31/573, A61K 31/704, A61K 31/475, A61P 35/00, A61P 35/04

(54) **COMBINATIONS FOR TREATING MULTIPLE MYELOMA**
KOMBINATIONEN ZUR BEHANDLUNG VON MULTIPLEM MYELOM
COMBINAISONS POUR LE TRAITEMENT DU MYÉLOME MULTIPLE

(30) Priority: 17.05.2002 US 380842 P; 06.11.2002 US 424600 P
(43) Date of publication of application: 16.02.2005
(60) Divisional application: 09004756.4
(73) Proprietor: CELGENE CORPORATION, Summit, NJ 07901 (US)
(72) Inventor: ZELDIS, Jerome B., Princeton, NJ 08540 (US)
(74) Representative: Jones Day
(86) International application number: PCT/US2003/015470
(87) International publication number: WO 2003/097052

(56) References cited:
- WO-A-02/059106
- WO-A2-01/70275
- US-B1- 6 316 471
- US-B1- 6 458 810
- DATABASE PHARMAML [Online] "Celgene's Revimid an orphan drug, says FDA" XP002369094 retrieved from STN Database accession no. 1659300 & MARKETLETTER OCTOBER 9, 2001, 9 October 2001 (2001-10-09),
- DATABASE NLDB [Online] "Celgene drug promises activity in solid tumors" XP002369095 retrieved from STN Database accession no. 2002:35280 & MARKETLETTER JUNE 18, 2001, 18 June 2001 (2001-06-18),
- FIGG WILLIAM D ET AL: "Inhibition of angiogenesis: Treatment options for patients with metastatic prostate cancer" INVESTIGATIONAL NEW DRUGS, vol. 20, no. 2, May 2002 (2002-05), pages 183-194, XP002369096 ISSN: 0167-6997
- RICHARDSON P G ET AL: "A phase 1 study of oral CC5013, an immunomodulatory thalidomide (thal) derivative, in patients with relapsed and refractory multiple myeloma (MM)" BLOOD, vol. 98, no. 11 Part 1, 16 November 2001 (2001-11-16), page 775a, XP009062262 & 43RD ANNUAL MEETING OF THE AMERICAN SOCIETY OF HEMATOLOGY, PART 1; ORLANDO, FLORIDA, USA; DECEMBER 07-11, 2001 ISSN: 0006-4971
- D'AMATO R J ET AL: "MECHANISM OF ACTION OF THALIDOMIDE AND 3-AMINOTHALIDOMIDE IN MULTIPLE MYELOMA" SEMINARS IN ONCOLOGY, W.B. SAUNDERS,, US, vol. 28, no. 6, December 2001 (2001-12), pages 597-601, XP008028089 ISSN: 0093-7754
- LENTZSCH S ET AL: "S-3-amino-phthalimido-glutarimide inhibits angiogenesis and growth of B-cell neoplasias in mice" CANCER RESEARCH, AMERICAN ASSOCIATION FOR CANCER RESEARCH, BALTIMORE, MD, US, vol. 62, 15 April 2002 (2002-04-15), pages 2300-2305, XP002959459 ISSN: 0008-5472
- WHITE D.J. ET AL: 'Addition of lenalidomide to melphalan in the treatment of newly diagnosed multiple myeloma: the National Cancer Institute of Canada Clinical Trials Group MY.11 trial.' CURRENT ONCOLOGY (TORONTO, ONT.) APR 2007 vol. 14, no. 2, April 2007, pages 61 - 65
- CHANAN-KHAN ASHER ALBAN ET AL: "Pegylated liposomal doxorubicin and immunomodulatory drug combinations in multiple myeloma: rationale and clinical experience." CLINICAL LYMPHOMA & MYELOMA APR 2007, vol. 7, April 2007 (2007-04), pages S163-S169,
- RICHARDSON P.G. ET AL: 'Management strategies for relapsed multiple myeloma' AMERICAN JOURNAL OF CANCER 2006 NZ vol. 5, no. 6, 2006, pages 393 - 409

## Description

### FIELD OF THE INVENTION

This invention relates to methods of treating, preventing and/or managing multiple myeloma by the administration of a immunomodulatory compound in combination with other therapeutics. In particular, the invention encompasses the use of specific combinations, or "cocktails " of drugs to treat multiple myeloma including multiple myeloma refractory to conventional therapy. The invention also relates to pharmaceutical compositions and dosing regimens.

### BACKGROUND OF THE INVENTION

### PATHOBIOLOGY OF CANCER AND OTHER DISEASES

Cancer is characterized primarily by an increase in the number of abnormal cells derived from a given normal tissue, invasion of adjacent tissues by these abnormal cells, or lymphatic or blood-borne spread of malignant cells to regional lymph nodes and to distant sites (metastasis). Clinical data and molecular biologic studies indicate that cancer is a multistep process that begins with minor preneoplastic changes, which may under certain conditions progress to neoplasia. The neoplastic lesion may evolve clonally and develop an increasing capacity for invasion, growth, metastasis, and heterogeneity, especially under conditions in which the neoplastic cells escape the host's immune surveillance. Roitt, I., Brostoff, J and Kale, D., Immunology, 17.1-17.12 (3rd ed., Mosby, St. Louis, Mo., 1993).

There is an enormous variety of cancers which are described in detail in the medical literature. Examples includes cancer of the lung, colon, rectum, prostate, breast, brain, and intestine. The incidence of cancer continues to climb as the general population ages, as new cancers develop, and as susceptible populations (*e*.*g*., people infected with AIDS or excessively exposed to sunlight) grow. A tremendous demand therefore exists for new methods and compositions that can be used to treat patients with cancer.

Many types of cancers are associated with new blood vessel formation, a process known as angiogenesis. Several of the mechanisms involved in tumor-induced angiogenesis have been elucidated. The most direct of these mechanisms is the secretion by the tumor cells of cytokines with angiogenic properties. Examples of these cytokines include acidic and basic fibroblastic growth factor (a,b-FGF), angiogenin, vascular endothelial growth factor (VEGF), and TNF-α. Alternatively, tumor cells can release angiogenic peptides through the production of proteases and the subsequent breakdown of the extracellular matrix where some cytokines are stored (*e*.*g*., b-FGF). Angiogenesis can also be induced indirectly through the recruitment of inflammatory cells (particularly macrophages) and their subsequent release of angiogenic cytokines (*e*.*g*., TNF-α, bFGF).

A variety of other diseases and disorders are also associated with, or characterized by, undesired angiogenesis. For example, enhanced or unregulated angiogenesis has been implicated in a number of diseases and medical conditions including, but not limited to, ocular neovascular diseases, choroidal neovascular diseases, retina neovascular diseases, rubeosis (neovascularization of the angle), viral diseases, genetic diseases, inflammatory diseases, allergic diseases, and autoimmune diseases. Examples of such diseases and conditions include, but are not limited to: diabetic retinopathy; retinopathy of prematurity; corneal graft rejection; neovascular glaucoma; retrolental fibroplasia; and proliferative vitreoretinopathy.

Accordingly, compounds that can control angiogenesis or inhibit the production of certain cytokines, including TNF-α, may be useful in the treatment and prevention of various diseases and conditions.

### METHODS OF TREATING CANCER

Current cancer therapy may involve surgery, chemotherapy, hormonal therapy and/or radiation treatment to eradicate neoplastic cells in a patient (see, for example, Stockdale, 1998, Medicine, vol. 3, Rubenstein and Federman, eds., Chapter 12, Section IV). Recently, cancer therapy could also involve biological therapy or immunotherapy. All of these approaches pose significant drawbacks for the patient. Surgery, for example, may be contraindicated due to the health of a patient or may be unacceptable to the patient. Additionally, surgery may not completely remove neoplastic tissue. Radiation therapy is only effective when the neoplastic tissue exhibits a higher sensitivity to radiation than normal tissue. Radiation therapy can also often elicit serious side effects. Hormonal therapy is rarely given as a single agent. Although hormonal therapy can be effective, it is often used to prevent or delay recurrence of cancer after other treatments have removed the majority of cancer cells. Biological therapies and immunotherapies are limited in number and may produce side effects such as rashes or swellings, flu-like symptoms, including fever, chills and fatigue, digestive tract.problems or allergic reactions.

With respect to chemotherapy, there are a variety of chemotherapeutic agents available for treatment of cancer. A majority of cancer chemotherapeutics act by inhibiting DNA synthesis, either directly, or indirectly by inhibiting the biosynthesis of deoxyribonucleotide triphosphate precursors, to prevent DNA replication and concomitant cell division. Gilman et al., Goodman and Gilman's: The Pharmacological Basis of Therapeutics, Tenth fid. (McGraw Hill, New York).

Despite availability of a variety of chemotherapeutic agents, chemotherapy has many drawbacks. Stockdale, Medicine, vol. 3, Rubenstein and Federman, eds., ch. 12, sect. 10, 1998. Almost all chemotherapeutic agents are toxic, and chemotherapy causes significant, and often dangerous side effects including severe nausea, bone marrow depression, and immunosuppression. Additionally, even with administration of combinations of chemotherapeutic agents, many tumor cells are resistant or develop resistance to the chemotherapeutic agents. In fact, those cells resistant to the particular chemotherapeutic agents used in the treatment protocol often prove to be resistant to other drugs, even if those agents act by different mechanism from those of the drugs used in the specific treatment. This phenomenon is referred to as pleiotropic drug or multidrug resistance. Because of the drug resistance, many cancers prove refractory to standard chemotherapeutic treatment protocols.

Other diseases or conditions associated with, or characterized by, undesired angiogenesis are also difficult to treat. However, some compounds such as protamine, hepain and steroids have been proposed to be useful in the treatment of certain specific diseases. Taylor et al., Nature 297:307 (1982); Folkman et al., Science 221:719 (1983); and U.S. Pat. Nos. 5,001,116 and 4,994,443. Thalidomide and certain derivatives of it have also been proposed for the treatment of such diseases and conditions. U.S. patent nos. 5,593,990, 5,629,327, 5,712,291, 6,071,948 and 6,114,355 to D'Amato.

Still, there is a significant need for safe and effective methods of treating, preventing and managing cancer and other diseases and conditions, particularly for diseases that are refractory to standard treatments, such as surgery, radiation therapy, chemotherapy and hormonal therapy, while reducing or avoiding the toxicities and/or side effects associated with the conventional therapies.

### IMIDS^{™}

A number of studies have been conducted with the aim of providing compounds that can safely and effectively be used to treat diseases associated with abnormal production of TNF-α *See, e*.*g*., Marriott, J.B., et al., Expert Opin. Biol. Ther. 1(4):1-8 (2001); G.W. Muller, et al., Journal of Medicinal Chemistry 39(17): 3238-3240 (1996); and G.W. Muller, et al., Bioorganic & Medicinal Chemistry Letters 8: 2669-2674 (1998). Some studies have focussed on a group of compounds selected for their capacity to potently inhibit TNF-α production by LPS stimulated PBMC. L.G. Corral, et al., Ann. Rheum. Dis. 58:(Suppl 1) 1107-1113 (1999). These compounds, which are referred to as IMiDs™ (Celgene Corporation) or Immunomodulatory Drugs, show not only potent inhibition of TNF-α but also marked inhibition of LPS induced monocyte IL1β and IL12 production. LPS induced IL6 is also inhibited by immunomodulatory compounds, *albeit* partially. These compounds are potent stimulators of LPS induced IL10. *Id.* Particular examples of IMiD^{™}s include, but are not limited to, the substituted 2-(2,6-dioxopiperidin-3-yl) phthalimides and substituted 2-(2,6-dioxopiperidin-3-yl)-1-oxoisoindoles described in United States Patent Nos. 6,281,230 and 6,316,471, both to G.W. Muller, et al.

### BRIEF DESCRIPTION OF FIGURE

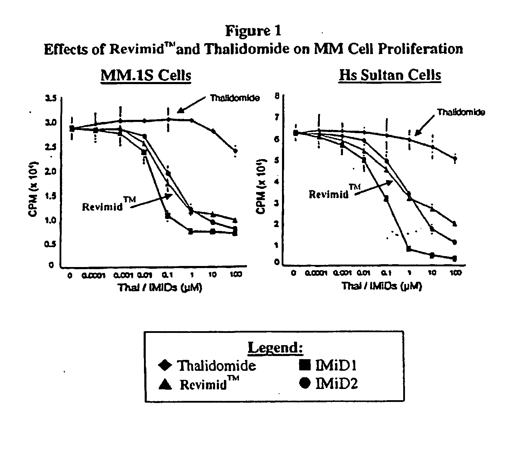

Figure 1 shows a comparison of the effects of 3-(4-amino-1-oxo-1,3-dihydroisoindol-2-yl)-piperidine-2,6-dione (Revimid^{™}) and thalidomide in inhibiting the proliferation of multiple myeloma (MM) cell lines in an *in vitro* study. The uptake of [³H]-thymidine by different MM cell lines (MM.1S, Hs Sultan, U266 and RPMI-8226) was measured as an indicator of the cell proliferation.

### DETAILED DESCRIPTION OF THE INVENTION

The invention encompasses a combination of a therapeutically effective amount of 3-(4-amino-l-oxo-1,3-dihydro-isoindol-2-yl)-piperidine-2,6-dione (REVIMID^{™}) and a therapeutically effective amount of a second active ingredient for use in treating multiple myeloma, (i) wherein the second active ingredient is dexamethasone; (ii) wherein the multiple myeloma is relapsed or refractory myeloma, and wherein the second active ingredient is a combination of doxorubicin and dexamethasone; or (iii) wherein the multiple myeloma is relapsed or refractory myeloma, and wherein the second active ingredient is a combination of doxorubicin, vincristin and dexamethasone. The invention further encompasses use of a therapeutically effective amount of Revimid^{™} and a therapeutically effective amount of a second active ingredient, in the preparation of a pharmaceutical composition for treating multiple myeloma, (i) wherein the second active ingredient is dexamethasone; (ii) wherein the multiple myeloma is relapsed or refractory myeloma, and wherein the second active ingredient is a combination of doxorubicin and dexamethasone; or (iii) wherein the multiple myeloma is relapsed or refractory myeloma, and wherein the second active ingredient is a combination of doxorubicin, vincristin and dexamethasone.

TNF-α is an inflammatory cytokine produced by macrophages and monocytes during acute inflammation. TNF-α is responsible for a diverse range of signaling events within cells. TNF-α may play a pathological role in cancer. Without being limited by theory, one of the biological effects exerted by the immunomodulatory compound of the invention is the reduction of synthesis of TNF-α. The immunomodulatory compound of the invention enhances the degradation of TNF-α mRNA.

Further, without being limited by theory, the immunomodulatory compound used in the invention may also be potent co-stimulators of T cells and increase cell proliferation dramatically in a dose dependent manner. The immunomodulatory compound may also have a greater co-stimulatory effect on the CD8+ T cell subset than on the CD4+ T cell subset. In addition, the compound preferably has anti-inflammatory properties, and efficiently co-stimulates T cells.

The immunomodulatory compound of the invention is 3-(4-amino-1-oxo-1,3-dihydro-isoindol-2-yl)-piperidine-2,6-dione. The compound can be obtained via standard, synthetic methods (*see e*.*g*., United States Patent No. 5,635,517). The compound is available from Celgene Corporation, Warren, NJ.
The compound 3-(4-amino-1-oxo-1,3-dihydro-isoindol-2-yl)-piperidine-2,6-dione (REVIMID^{™}) has the following chemical structure:

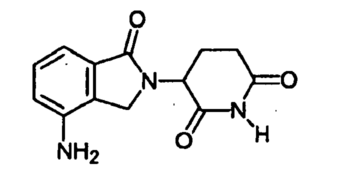

The compound of the invention can either be commercially purchased or prepared according to the methods described in the patents or patent publications disclosed herein.

As used herein and unless otherwise indicated, the term "pharmaceutically acceptable salt" encompasses non-toxic acid and base addition salts of the compound to which the term refers. Acceptable non-toxic acid addition salts include those derived from organic and inorganic acids or bases know in the art, which include, for example, hydrochloric acid, hydrobromic acid, phosphoric acid, sulfuric acid, methanesulphonic acid, acetic acid, tartaric acid, lactic acid, succinic acid, citric acid, malic acid, maleic acid, sorbic acid, aconitic acid, salicylic acid, phthalic acid, embolic acid, enanthic acid, and the like.

Compounds that are acidic in nature are capable of forming salts with various pharmaceutically acceptable bases. The bases that can be used to prepare pharmaceutically acceptable base addition salts of such acidic compounds are those that form non-toxic base addition salts, *i*.*e*., salts containing pharmacologically acceptable cations such as, but not limited to, alkali metal or alkaline earth metal salts and the calcium, magnesium, sodium or potassium salts in particular. Suitable organic bases include, but are not limited to, N,N-dibenzylethylenediamine, chloroprocaine, choline, diethanolamine, ethylenediamine, meglumaine (N-methylglucamine), lysine, and procaine.

As used herein and unless otherwise indicated, the term "prodrug" means a derivative of a compound that can hydrolyze, oxidize, or otherwise react under biological conditions (*in vitro* or *in vivo*) to provide the compound. Examples are derivatives of the immunomodulatory compound that comprise biohydrolyzable moieties such as biohydrolyzable amides, biohydrolyzable esters, biohydrolyzable carbamates, biohydrolyzable carbonates, biohydrolyzable ureides, and biohydrolyzable phosphate analogues. Other examples of prodrugs include derivatives of the immunomodulatory compound of the invention that comprise -NO, -NO₂, -ONO, or -ONO₂ moieties. Prodrugs can typically be prepared using well-known methods, such as those described in 1 Burger's Medicinal Chemistry and Drug Discovery, 172-178, 949-982 (Manfred E. Wolff ed., 5th ed. 1995), and Design of Prodrugs (H. Bundgaard ed., Elselvier, New York 1.985).

As used herein and unless otherwise indicated, the terms "biohydrolyzable amide," "biohydrolyzable ester," "biohydrolyzable carbamate," "biohydrolyzable carbonate," "biohydrolyzable ureide," "biohydrolyzable phosphate" mean an amide, ester, carbamate, carbonate, ureide, or phosphate, respectively, of a compound that either: 1) does not interfere with the biological activity of the compound but can confer upon that compound advantageous properties *in vivo*, such as uptake, duration of action, or onset of action; or 2) is biologically inactive but is converted *in vivo* to the biologically active compound. Examples of biohydrolyzable esters include, but are not limited to, lower alkyl esters, lower acyloxyalkyl esters (such as acetoxylmethyl, acetoxyethyl, aminocarbonyloxymethyl, pivaloyloxymethyl; and pivaloyloxyethyl esters), lactonyl esters (such as phthalidyl and thiophthalidyl esters), lower alkoxyacyloxyalkyl esters (such as methoxycarbonyloxymethyl, ethoxycarbonyloxyethyl and isopropoxycarbonyloxyethyl esters), alkoxyalkyl esters, choline esters, and acylamino alkyl esters (such as acetamidomethyl esters). Examples of biohydrolyzable amides include, but are not limited to, lower alkyl amides, α-amino acid amides, alkoxyacyl amides, and alkylaminoalkylcarbonyl amides. Examples of biohydrolyzable carbamates include, but are not limited to, lower alkylamines, substituted ethylenediamines, amino acids, hydroxyalkylamines, heterocyclic and heteroaromatic amines, and polyether amines.

As used herein and unless otherwise indicated, the term "stereomerically pure" means a composition that comprises one stereoisomer of a compound and is substantially free of other stereoisomers of that compound. For example, a stereomerically pure composition of a compound having one chiral center will be substantially free of the opposite enantiomer of the compound. A stereomerically pure composition of a compound having two chiral centers will be substantially free of other diastereomers of the compound. A typical stereomerically pure compound comprises greater than about 80% by weight of one stereoisomer of the compound and less than about 20% by weight of other stereoisomers of the compound, more preferably greater than about 90% by weight of one stereoisomer of the compound and less than about 10% by weight of the other stereoisomers of the compound, even more preferably greater than about 95% by weight of one stereoisomer of the compound and less than about 5% by weight of the other stereoisomers of the compound, and most preferably greater than about 97% by weight of one stereoisomer of the compound and less than about 3% by weight of the other stereoisomers of the compound. As used herein and unless otherwise indicated, the term "stereomerically enriched" means a composition that comprises greater than about 60% by weight of one stereoisomer of a compound, preferably greater than about 70% by weight, more preferably greater than about 80% by weight of one stereoisomer of a compound. As used herein and unless otherwise indicated, the term "enantiomerically pure" means a stereomerically pure composition of a compound having one chiral center Similarly, the term "stereomerically enriched" means a stereomerically enriched composition of a compound having one chiral center.

It should be noted that if there is a discrepancy between a depicted structure and a name given that structure, the depicted structure is to be accorded more weight. In addition, if the stereochemistry of a structure or a portion of a structure is not indicated with, for example, bold or dashed lines, the structure or portion of the structure is to be interpreted as encompassing all stereoisomers of it.

The immunomodulatory compound can be combined with other pharmacologically active compounds ("second active agents") in uses and compositions of the invention. It is believed that certain combinations work synergistically in the treatment of particular types of cancer. The immunomodulatory compound can also work to alleviate adverse effects associated with certain second active agents, and some second active agents can be used to alleviate adverse effects associated with the immunomodulatory compound.

One or more second active ingredients or agents can be used in the uses and compositions of the invention together with the immunomodulatory compound.

As used herein, unless otherwise specified, the term "treating" refers to the administration of the immunomodulatory compound or other additional active agent after the onset of symptoms of the particular disease or disorder. As used herein and unless otherwise indicated, the term "managing" encompasses preventing the recurrence of the particular disease or disorder in a patient who had suffered from it, and/or lengthening the time a patient who had suffered from the disease or disorder remains in remission.

This invention encompasses the uses disclosed herein for treating patients who have been previously treated for multiple myeloma, but are non-responsive to standard therapies, as well those who have not previously been treated. The invention also encompasses the uses disclosed herein for treating patients regardless of patient's age.

The uses encompassed by this invention comprise the preparation of a medicament for the administering immunomodulatory compound of the invention, or a pharmaceutically acceptable salt, solvate or hydrate thereof, to a patient (*e*.*g*., a human) suffering from multiple myeloma.

In one embodiment of the invention, the immunomodulatory compound of the invention can be prepared to be administered orally and in single or divided daily doses in an amount of from about 0.10 to about 150 mg/day.

In a particular embodiment, 3-(4-anaino-1-oxo -1,3-dihydro-isoiadol-2-yl)-pipexidine-2,6-dione (Revimid^{™}) may be administered initially in an amount of 5 mg/day and the dose can be escalated every week to 10, 20, 25, 30 and 50 mg/day.

### COMBINATION THERAPY WITH A SECOND ACTIVE AGENT

Specific methods disclosed herein comprise administering the immunomodulatory compound, or a pharmaceutically acceptable salt, solvate, hydrate, stereoisomer, clathrate, or prodrug thereof, in combination with one or more second active agents.

Administration of the immunomodulatory compound and the second active agents to a patient can occur simultaneously or sequentially by the same or different routes of administration. The suitability of a particular route of administration employed for a particular active agent will depend on the active agent itself (*e*.*g*., whether it can be administered orally without decomposing prior to centering the blood stream. A preferred route of administration for the immunomodulatory compound is orally. Preferred routes of administration for the second active agents or ingredients are known to those of ordinary skill in the art. *See, e*.*g*., Physicians' Desk Reference, 1755-1760 (56th ed., 2002).

In one embodiment, the second active agent is administered intravenously or subcutaneously and once or twice daily in an amount of from about 1 to about 1000 mg, from about 5 to about 500 mg, from about 10 to about 350 mg, or from about 50 to about 200 mg. The specific amount of the second active agent will depend on the specific agent used, the type of disease being treated or managed, the severity and stage of disease, and the amount(s) of immunomodulatory compounds and any optional additional active agents concurrently administered to the patient.

In a specific embodiment of the invention, Revimid^{™} is administered to patients with relapsed or refractory multiple myeloma in an amount of about 15 mg/d twice a day or about 30 mg/d four times a day in a combination with dexamethasone.

In another embodiment, the immunomodulatory compound is used in the preparation of a medicament for administration to patients with various types or stages of multiple myeloma in combination with dexamethasone.

In another embodiment, an immunomodulatory compound is administered to patients with relapsed or refractory multiple myeloma in combination with doxorubicin (Doxil^{®}), vincristine and/or dexamethasone (Decadron^{®}).

In a specific embodiment of the invention, Revimid^{™} in combination with doxorubicin and dexamethasone or in combination with vincristin and dexamethasone is used for the preparation of a pharmaceutical composition for treating relapsed or refractory myeloma.

This disclosure also encompasses a method of increasing the dosage of an anti-cancer drug or agent that can be safely and effectively administered to a patient, which comprises administering to a patient (*e*.*g*., a human) the immunomodulatory compound, or a pharmaceutically acceptable salt, solvate or hydrate thereof. The administration of the immunomodulatory compound alleviates or reduces adverse effects which are of such severity that it would otherwise limit the amount of anti-cancer drug.

In one embodiment, an immunomodulatory compound can be administered orally and daily in an amount of from about 0.1 to about 150 mg, and preferably from about 1 to about 50 mg, more preferably from about 2 to about 25 mg prior to, during, or after the occurrence of the adverse effect associated with the administration of an anti-cancer drug to a patient.

This disclosure encompasses a method of treating, preventing and/or managing multiple myeloma, which comprises administering an immunomodulatory compound, or a pharmaceutically acceptable salt, solvate or hydrate thereof, in conjunction with (*e*.*g*. before, during, or after) conventional therapy including, but not limited to, surgery, immunotherapy, biological therapy, radiation therapy, for other non-drug based therapy presently used to treat, prevent or manage multiple myeloma. The combined use of the immunomodulatory compound and conventional therapy may provide a unique treatment regimen that is unexpectedly effective in certain patients. Without being limited by theory, it is believed that the immunomodulatory compound may provide additive or synergistic effects when given concurrently with conventional therapy.

As discussed elsewhere herein, the disclosure encompasses a method of reducing, treating and/or preventing adverse or undesired effects associated with conventional therapy including, but not limited to, surgery, chemotherapy, radiation therapy, hormonal therapy, biological therapy and immunotherapy. The immunomodulatory compound and other active ingredients can be administered to a patient prior to, during, or after the occurrence of the adverse effect associated with conventional therapy.

In one embodiment, the immunomodulatory compound can be administered in an amount of from about 0.1 to about 150 mg, and preferably from about 1 to about 25 mg, more preferably from about 2 to about 10 mg orally and daily alone, or in combination with a second active agent disclosed herein, prior to, during, or after the use of conventional therapy.

### USE WITH TRANSPLANTATION THERAPY

The compounds disclosed herein can be used to reduce the risk of Graft Versus Host Disease (GVHD). Therefore, the disclosure encompasses a method of treating, preventing and/or managing multiple myeloma, which comprises administering the immunomodulatory compound, or a pharmaceutically acceptable salt, solvate or hydrate thereof, in conjunction with transplantation therapy.

As those of ordinary skill in the art are aware, the treatment of multiple myeloma is often based on the stages and mechanism of the disease. For example, as inevitable leukemic transformation develops in certain stages of multiple myeloma, transplantation of peripheral blood stem cells, hematopoietic stem cell preparation or bone marrow may be necessary. The combined use of the immunomodulatory compound and transplantation therapy provides a unique and unexpected synergism. In particular, the immunomodulatory compound exhibits immunomodulatory activity that may provide additive or synergistic effects when given concurrently with transplantation therapy in patients with cancer.

The immunomodulatory compound can work in combination with transplantation therapy reducing complications associated with the invasive procedure of transplantation any risk of GVHD. This disclosure encompasses a method of treating, preventing and/or managing multiple myeloma which comprises administering to a patient (*e*.*g*., a human) an immunomodulatory compound, or a pharmaceutically acceptable salt, solvate or hydrate thereof, before, during, or after the transplantation of umbilical cord blood placental blood, peripheral blood stem cell, hematopoietic stem cell preparation or bone marrow. Examples of stem cells suitable for use in the methods described herein are disclosed in U.S. provisional patent application no. 60/372,348, filed April 12,2002 by R. Hariri et al..

In one embodiment of this method, an immunomodulatory compound is administered to patients with multiple myeloma before, during, or after the transplantation of autologous peripheral blood progenitor cell.

In another embodiment, an immunomodulatory compound is administered to patients with relapsing multiple, myeloma after the stem cell transplantation.

In another embodiment, an immunomodulatory compound and prednisone are administered as maintenance therapy to patients with multiple myeloma following the transplantation of autologous stem cell.

In another embodiment, an immunomodulatory compound and dexamethasone are administered as salvage therapy for low risk post transplantation to patients with multiple myeloma.

In another embodiment, an immunomodulatory compound and dexamethasone are administered as maintenance therapy to patients with multiple myeloma following the transplantation of autologous bone marrow.

### CYCLING THERAPY

In certain embodiments, the prophylactic or therapeutic agents are cyclically administered to a patient Cycling therapy involves the administration of an active agent for a period of time, followed by a rest for a period of time, and repeating this sequential administration. Cycling therapy can reduce the development of resistance to one or more of the therapies, avoid or reduce the side effects of one of the therapies, and/or improves the efficacy of the treatment.

Consequently, in one embodiment, the immunomodulatory compound is administered daily in a single or divided doses in a four to six week cycle with a rest period of about a week or two weeks. The disclosure further allows the frequency, number, and length of dosing cycles to be increased. Thus, another embodiment encompasses the administration of the immunomodulatory compound for more cycles than are typical when it is administered alone. In yet another embodiment, the immunomodulatory compound is administered for a greater number of cycles that would typically cause dose-limiting toxicity in a patient to whom a second active ingredient is not also being administered.

In one embodiment, the immunomodulatory compound is administered daily and continuously for three or four weeks at a dose of from about 0.1 to about 150 mg/d followed by a break of one or two weeks. In a particular embodiment, Revimid^{™} is administered in an amount of about 5, 10, or 25mg/day, preferably in an amount of about 10 mg/day for three to four weeks, followed by one week or two weeks of rest in a four or six week cycle.

In one embodiment the immunomodulatory compound and a second active ingredient are administered orally, with administration of the immunomodulatory compound occurring 30 to 60 minutes prior to a second active ingredient, during a cycle of four to six weeks. In another embodiment, the combination of the immunomodulatory compound of the invention and a second active ingredient is administered by intravenous infusion over about 90 minutes every cycle. In a certain embodiment, one cycle comprises the administration of from about 10 to about 25 mg/day of Revimid^{™} and from about 50 to about 200 mg/m²/day of a second active ingredient daily for three to four weeks and then one or two weeks of rest. Typically, the number of cycles during which the combinatorial treatment is administered to a patient will be from about one to about 24 cycles, more typically from about two to about 16 cycles, and even more typically from about four to about three cycles.

### PHARMACEUTICAL COMPOSITIONS AND DOSAGE FORMS

Pharmaceutical compositions can be used in the preparation of individual single unit dosage forms. Pharmaceutical compositions and dosage forms comprise an immunomodulatory compound; or a pharmaceutically acceptable salt, solvate or hydrate thereof. Pharmaceutical compositions and dosage forms can further comprise one or more excipients.

Pharmaceutical compositions and dosage forms comprise the active ingredients disclosed herein (*e.g.*, the immunomodulatory compound and a second active agent).

Single unit dosage forms of the invention are suitable for oral, mucosal (*e*.*g*., nasal, sublingual, vaginal, buccal, or rectal), parenteral (*e*.*g*., subcutaneous, intravenous, bolus injection, intramuscular, or intraarterial), topical (*e*.*g*., eye drops or other ophthalmic preparations), transdermal or transcutaneous administration to a patient. Examples of dosage forms include, but are not limited to: tablets; caplets; capsules, such as soft elastic gelatin capsules; cachets; troches; lozenges; dispersions; suppositories; powders; aerosols (*e*.*g*., nasal sprays or inhalers); gels; liquid dosage forms suitable for oral or mucosal administration to a patient, including suspensions (*e*.*g.*, aqueous or non-aqueous liquid suspensions, oil-in-water emulsions, or a water-in-oil liquid emulsions), solutions, and elixirs; liquid dosage forms suitable for parenteral administration to a patient; eye drops or other ophthalmic preparations suitable for topical administration; and sterile solids (*e*.*g*., crystalline or amorphous solids) that can be reconstituted to provide liquid dosage forms suitable for parenteral administration to a patient.

The composition, shape, and type of dosage forms will typically vary depending on their use. For example, a dosage form used in the acute treatment of a disease may contain larger amounts of one or more of the active ingredients it comprises than a dosage form used in the chronic treatment of the same disease. Similarly, a parenteral dosage form may contain smaller amounts of one or more of the active ingredients it comprises than an oral dosage form used to treat the same disease. These and other ways in which specific dosage forms disclosed herein will vary from one another will be readily apparent to those skilled in the art. *See, e.g.,* Remington's Pharmaceutical Sciences, 18th ed., Mack Publishing, Easton PA (1990).

Typical pharmaceutical compositions and dosage forms comprise one or more excipients. Suitable excipients are well known to those skilled in the art of pharmacy, and non-limiting examples of suitable excipients are provided herein. Whether a particular excipient is suitable for incorporation into a pharmaceutical composition or dosage form depends on a variety of factors well known in the art including, but not limited to, the way in which the dosage form will be administered to a patient. For example, oral dosage forms such as tablets may contain excipients not suited for use in parenteral dosage forms. The suitability of a particular excipient may also depend on the specific active ingredients in the dosage form. For example, the decomposition of some active ingredients may be accelerated by some excipients such as lactose, or when exposed to water. Active ingredients that comprise primary or secondary amines are particularly susceptible to such accelerated decomposition. Consequently, this disclosure encompasses pharmaceutical compositions and dosage forms that contain little, if any, lactose other mono- or disaccharides. As used herein, the term "lactose-free" means that the amount of lactose present, if any, is insufficient to substantially increase the degradation rate of an active ingredient.

Lactose-free compositions can comprise excipients that are well known in the art and are listed, for example, in the *U.S. Pharmacopeia* (USP) 25-NF20 (2002). In general, lactose-free compositions comprise active ingredients, a binder/filler, and a lubricant in pharmaceutically compatible and pharmaceutically acceptable amounts. Preferred lactose-free dosage forms comprise active ingredients, microcrystalline cellulose, pre-gelatinized starch, and magnesium stearate.

This disclosure further encompasses anhydrous pharmaceutical compositions and dosage forms comprising active ingredients, since water can facilitate the degradation of some compounds. For example, the addition of water (*e.g.*, 5%) is widely accepted in the pharmaceutical arts as a means of simulating long-term storage in order to determine characteristics such as shelf-life or the stability of formulations over time. *See, e.g*., Jens T. Carstensen, Drug Stability: Principles & Practice, 2d. Ed., Marcel Dekker, NY, NY, 1995, pp. 379-80. In effect, water and heat accelerate the decomposition of some compounds. Thus, the effect of water on a formulation can be of great significance since moisture and/or humidity are commonly encountered during manufacture, handling, packaging, storage, shipment, and use of formulations.

Anhydrous pharmaceutical compositions and dosage forms can be prepared using anhydrous or low moisture containing ingredients and low moisture or low humidity conditions. Pharmaceutical compositions and dosage forms that comprise lactose and at least one active ingredient that comprises a primary or secondary amine are preferably anhydrous if substantial contact with moisture and/or humidity during manufacturing, packaging, and/or storage is expected.

An anhydrous pharmaceutical composition should be prepared and stored such that its anhydrous nature is maintained. Accordingly, anhydrous compositions are preferably packaged using materials know to prevent exposure to water such that they can be included in suitable formulary kits. Examples of suitable packaging include, but are not limited to, hermetically sealed foils, plastics, unit dose containers (*e*.*g*., vials), blister packs, and strip packs.

The disclosure further encompasses pharmaceutical compositions and dosage forms that comprise one or more compounds that reduce the rate by which an active ingredient will decompose. Such compounds, which are referred to herein as "stabilizers," include, but are not limited to, antioxidants such as ascorbic acid, pH buffers, or salt buffers.

Like the amounts and types of excipients, the amounts and specific types of active ingredients in a dosage form may differ depending on factors such as, but not limited to, the route by which it is to be administered to patients. However, typical dosage forms comprise the immunomodulatory compound or a pharmaceutically acceptable salt, solvate or hydrate thereof in an amount of from about 0:10 to about 150 mg Typical dosage forms comprise the immunomodulatory compound or a pharmaceutically acceptable salt, solvate or hydrate thereof in an amount of about 0.1, 1, 2, 5, 7.5, 10, 12.5, 15, 17.5, 20, 25, 50, 100, 150 or 200 mg. In one embodiment, a preferred dosage form comprises 3-(4-amino-1-oxo-1,3-dihydro-isoindol-2-yl)-piperidine-2,6-dione (Rovimid^{™}) in an amount of about 5, 10, 25 or 50mg. Typical dosage forms comprise the second active ingredient in an amount of 1 to about 1000 mg, from about 5 to about 500 mg, from about 10 to about 350 mg, or from about 50 to about 200 mg. Of course, the specific amount of the anti-cancer drug will depend on the specific agent used, and the amount(s) of the immunomodulatory compound and any optional additional active agents concurrently administered to the patient.

### ORAL DOSAGE FORMS

Pharmaceutical compositions that are suitable for oral administration can be presented as discrete dosage forms, such as, but are not limited to, tablets (*e.g.,* chewable tablets), caplets, capsules, and liquids (*e*.*g*., flavored syrups). Such dosage forms contain predetermined amounts of active ingredients, and may be prepared by methods of pharmacy well known to those skilled in the art. *See generally,* Remington's Pharmaceutical Sciences, 18th ed., Mack Publishing, Easton PA (1990).

Typical oral dosage forms are prepared by combining the active ingredients in an intimate admixture with at least one excipient according to conventional pharmaceutical compounding techniques. Excipients can take a wide variety of forms depending on the form of preparation desired for administration. For example, excipients suitable for use in oral liquid or aerosol dosage forms include, but are not limited to, water, glycols, oils, alcohols, flavoring agents, preservatives, and coloring agents. Examples of excipients suitable for use in solid oral dosage forms (*e*.*g*., powders, tablets, capsules, and caplets) include, but are not limited to, starches, sugars, micro-crystalline cellulose, diluents, granulating agents, lubricants, binders, and disintegrating agents.

Because of their ease of administration, tablets and capsules represent the most advantageous oral dosage unit forms, in which case solid excipients are employed. If desired, tablets can be coated by standard aqueous or nonaqueous techniques. Such dosage forms can be prepared by any of the methods of pharmacy. In general, pharmaceutical compositions and dosage forms are prepared by uniformly and intimately admixing the active ingredients with liquid carriers, finely divided solid carriers, or both, and then shaping the product into the desired presentation if necessary.

For example, a tablet can be prepared by compression or molding. Compressed tablets can be prepared by compressing in a suitable machine the active ingredients in a free-flowing form such as powder or granules, optionally mixed with an excipient. Molded tablets can be made by molding in a suitable machine a mixture of the powdered compound moistened with an inert liquid diluent.

Examples of excipients that can be used in oral dosage forms include, but are not limited to, binders, fillers, disintegrants, and lubricants. Binders suitable for use in pharmaceutical compositions and dosage forms include, but are not limited to, corn starch, potato starch, or other starches, gelatin, natural and synthetic gums such as acacia, sodium alginate, alginic acid, other alginates, powdered tragacanth, guar gum, cellulose and its derivatives (*e.g.*, ethyl cellulose, cellulose acetate, carboxymethyl cellulose calcium, sodium carboxymethyl cellulose), polyvinyl pyrrolidone, methyl cellulose, pre-gelatinized starch, hydroxypropyl methyl cellulose, (*e.g.*, Nos. 2208, 2906, 2910), microcrystalline cellulose, and mixtures thereof.

Suitable forms of microcrystalline cellulose include, but are not limited to, the materials sold as AVICEL-PH-101, AVICEL-PH-103 AVICEL RC-581, AVICEL-PH-105 (available from FMC Corporation, American Viscose Division, Avicel Sales, Marcus Hook, PA), and mixtures thereof. An specific binder is a mixture of microcrystalline cellulose and sodium carboxymethyl cellulose sold as AVICEL RC-581. Suitable anhydrous or low moisture excipients or additives include AVICEL-PH-103™ and Starch 1500 LM.

Examples of fillers suitable for use in the pharmaceutical compositions and dosage forms disclosed herein include, but are not limited to, talc, calcium carbonate (*e.g.*, granules or powder), microcrystalline cellulose, powdered cellulose, dextrates, kaolin, mannitol, silicic acid, sorbitol, starch, pre-gelatinized starch, and mixtures thereof. The binder or filler in pharmaceutical compositions of the invention is typically present in from about 50 to about 99 weight percent of the pharmaceutical composition or dosage form.

Disintegrants are used in the compositions to provide tablets that disintegrate when exposed to an aqueous environment Tablets that contain too much disintegrant may disintegrate in storage, while those that contain too little may not disintegrate at a desired rate or under the desired conditions. Thus, a sufficient amount of disintegrant that is neither too much nor too little to detrimentally alter the release of the active ingredients should be used to form solid oral dosage forms of the invention. The amount of disintegrant used varies based upon the type of formulation, and is readily discernible to those of ordinary skill in the art. Typical pharmaceutical compositions comprise from about 0.5 to about 15 weight percent of disintegrant, preferably from about 1 to about 5 weight percent of disintegrant.

Disintegrants that can be used in pharmaceutical compositions and dosage forms include, but are not limited to, agar-agar, alginic acid, calcium carbonate, microcrystalline cellulose, croscarmellose sodium, crospovidone, polacrilin potassium, sodium starch glycolate, potato or tapioca starch, other starches, pre-gelatinized starch, other starches, clays; other algins, other celluloses, gums, and mixtures thereof.

Lubricants that can be used in pharmaceutical compositions and dosage forms include, but are not limited to, calcium stearate, magnesium stearate, mineral oil, light mineral oil, glycerin, sorbitol, mannitol, polyethylene glycol, other glycols, stearic acid, sodium lauryl sulfate, talc, hydrogenated vegetable oil (*e.g.*, peanut oil, cottonseed oil, sunflower oil, sesame oil, olive oil, corn oil, and soybean oil), zinc stearate, ethyl oleate, ethyl laureate, agar, and mixtures thereof. Additional lubricants include, for example, a syloid silica gel (AEROSIL200, manufactured by W.R. Grace Co. of Baltimore, MD), a coagulated aerosol of synthetic silica (marketed by Degussa Co. of Plano, TX), CAB-O-SIL (a pyrogenic silicon dioxide product sold by Cabot Co. of Boston, MA), and mixtures thereof. If used at all, lubricants are typically used in an amount of less than about 1 weight percent of the pharmaceutical compositions or dosage forms into which they are incorporated.

A preferred solid oral dosage form comprises the immunomodulatory compound, anhydrous lactose, microcrystalline cellulose, polyvinylpyrrolidone, stearic acid, colloidal anhydrous silica, and gelatin.

### DELAYED RELEASE DOSAGE FORMS

Active ingredients can be administered by controlled release means or by delivery devices that are well known to those of ordinary skill in the art. Examples include, but are not limited to, those described in U.S. Patent Nos.: 3,845,770; 3,916,899; 3,536,809; 3,598,123; and 4,008,719, 5,674,533, 5,059,595, 5,591,767, 5,120,548, 5,073,543, 5,639,476, 5,354,556, and 5,733,566. Such dosage forms can be used to provide slow or controlled-release of one or more active ingredients using, for example, hydropropylmethyl cellulose, other polymer matrices, gels, permeable membranes, osmotic systems, multilayer coatings, microparticles, liposomes, microspheres, or a combination thereof to provide the desired release profile in varying proportions. Suitable controlled-release formulations known to those of ordinary skill in the art, including those described herein, can be readily selected for use with the active ingredients. The disclosure thus encompasses single unit dosage forms suitable for oral administration such as, but not limited to, tablets, capsules, gelcaps, and caplets that are adapted for controlled-release.

All controlled-release pharmaceutical products have a common goal of improving drug therapy over that achieved by their non-controlled counterparts. Ideally, the use of an optimally designed controlled-release preparation in medical treatment is characterized by a minimum of drug substance being employed to cure or control the condition in a minimum amount of time. Advantages of controlled-release formulations include extended activity of the drug, reduced dosage frequency, and increased patient compliance. In addition, controlled-release formulations can be used to affect the time of onset of action or other characteristics, such as blood levels of the drug, and can thus affect the occurrence of side (*e.g.*, adverse) effects.

Most controlled-release formulations are designed to initially release an amount of drug (active ingredient) that promptly produces the desired therapeutic effect, and gradually and continually release of other amounts of drug to maintain this level of therapeutic or prophylactic effect over an extended period of time. In order to maintain this constant level of drug in the body, the drug must be released from the dosage form at a rate that will replace the amount of drug being metabolized and excreted from the body. Controlled-release of an active ingredient can be stimulated by various conditions including, but not limited to, pH, temperature, enzymes, water, or other physiological conditions or compounds.

### PARENTERAL DOSAGE FORMS

Parenteral dosage forms can be administered to patients by various routes including, but not limited to, subcutaneous, intravenous (including bolus injection), intramuscular, and intraarterial. Because their administration typically bypasses patients' natural defenses against contaminants, parenteral dosage forms are preferably sterile or capable of being sterilized prior to administration to a patient. Examples of parenteral dosage forms include, but are not limited to, solutions ready for injection, dry products ready to be dissolved or suspended in a pharmaceutically acceptable vehicle for injection, suspensions ready for injection, and emulsions.

Suitable vehicles that can be used to provide parenteral dosage forms are well know to those skilled in the art. Examples include, but are not limited to: Water for Injection USP; aqueous vehicles such as, but not limited to, Sodium Chloride Injection, Ringer's Injection, Dextrose Injection, Dextrose and Sodium Chloride Injection, and Lactated Ringer's Injection; water-miscible vehicles such as, but not limited to, ethyl alcohol, polyethylene glycol, and polypropylene glycol; and non-aqueous vehicles such as, but not limited to, corn oil, cottonseed oil, peanut oil, sesame oil, ethyl oleate, isopropyl myristate, and benzyl benzoate.

Compounds that increase the solubility of one or more of the active ingredients disclosed herein can also be incorporated into the parenteral dosage forms. For example, cyclodextrin and its derivatives can be used to increase the solubility of an immunomodulatory compound and its derivatives. *See, e*.*g*., U.S. Patent No. 5,134,127.

### TOPICAL AND MUCOSAL DOSAGE FORMS

Topical and mucosal dosage forms include, but are not limited to, sprays, aerosols, solutions, emulsions, suspensions, eye drops or other ophthalmic preparations, or other forms known to one of skill in the art. *See, e.g.,* Remington's Pharmaceutical Sciences, 16th and 18th eds., Mack Publishing, Easton PA (1980 & 1990); and Introduction to Pharmaceutical Dosage Forms, 4th ed., Lea & Febiger, Philadelphia (1985). Dosage forms suitable for treating mucosal tissues within the oral cavity can be formulated as mouthwashes or as oral gels.

Suitable excipients (*e*.*g*., carriers and diluents) and other materials that can be used to provide topical and mucosal dosage forms are well known to those skilled in the pharmaceutical arts, and depend on the particular tissue to which a given pharmaceutical composition or dosage form will be applied. With that fact in mind, typical excipients include, but are not limited to, water, acetone, ethanol, ethylene glycol, propylene glycol, butane-1,3-diol, isopropyl myristate, isopropyl palmitate, mineral oil, and mixtures thereof to form solutions, emulsions or gels, which are non-toxic and pharmaceutically acceptable. Moisturizers or humectants can also be added to pharmaceutical compositions and dosage forms if desired. Examples of such additional ingredients are well known in the art. *See, e*.*g*., Remington's Pharmaceutical Sciences, 16th and 18th eds., Mack Publishing, Easton PA (1980 & 1990).

The pH of a pharmaceutical composition or dosage form may also be adjusted to improve delivery of one or more active ingredients. Similarly, the polarity of a solvent carrier, its ionic strength, or tonicity can be adjusted to improve delivery. Compounds such as stearates can also be added to pharmaceutical compositions or dosage forms to advantageously alter the hydrophilicity or lipophilicity of one or more active ingredients so as to improve delivery. In this regard, stearates can serve as a lipid vehicle for the formulation, as an emulsifying agent or surfactant, and as a delivery-enhancing or penetration-enhancing agent. Different salts, hydrates or solvates of the active ingredients can be used to further adjust the properties of the resulting composition.

### 5.4.5 KITS

Typically, active ingredients are preferably not administered to a patient at the same time or by the same route of administration. This disclosure therefore encompasses kits which, when used by the medical practitioner, can simplify the administration of appropriate amounts of active ingredients to a patient

A typical kit comprises a dosage form of an immunomodulatory compound, or a pharmaceutically acceptable salt, solvate or hydrate, thereof.

Kits can further comprise devices that are used to administer the active ingredients. Examples of such devices include, but are not limited to, syringes, drip bags, patches, and inhalers.

Kits can further comprise cells or blood for transplantation as well as pharmaceutically acceptable vehicles that can be used to administer one or more active ingredients. For example, ifan active ingredient is provided in a solid form that must be reconstituted for parenteral administration, the kit can comprise a sealed container of a suitable vehicle in which the active ingredient can be dissolved to form a particulate-free sterile solution that is suitable for parenteral administration. Examples of pharmaceutically acceptable vehicles include, but are not limited to: Water for Injection USP; aqueous vehicles such as, but not limited to, Sodium Chloride Injection, Ringer's Injection, Dextrose Injection, Dextrose and Sodium Chloride Injection, and Lactated Ringer's Injection; water-miscible vehicles such as, but not limited to, ethyl alcohol, polyethylene glycol, and polypropylene glycol; and non-aqueous vehicles such as, but not limited to, corn oil, cottonseed oil, peanut oil, sesame oil, ethyl oleate, isopropyl myristate, and benzyl benzoate.

### COMPARATIVE EXAMPLES

Certain embodiments of the invention are illustrated by the following non-limiting examples.

### MODULATION OF CYTOKINE PRODUCTION

A series of non-clinical pharmacology and toxicology studies have been performed to support the clinical evaluation of an immunomodulatory compound in human subjects. These studies were performed in accordance with internationally recognized guidelines for study design and in compliance with the requirements of Good Laboratory Practice (GLP), unless otherwise noted.

Inhibition of TNF-α production following LPS-stimulation of human PBMC and human whole blood by 4-(amino)-2-(2,6-dioxo(3-piperidyl))-isoindoline-1,3-dione (Actimid™), 3-(4-amino-1-oxo-1,3-dihydro-isoindol-2-yl)-piperidine-2,6-dione and thalidomide (Revimid^{™}) was investigated *in vitro* (Muller et al., Bioorg. Med. Chem. Lett. 9:1625-1630, 1999). The IC₅₀'s of 4-(amino)-2-(2,6-dioxo(3-piperidyl))-islindoline-1,3-dione for inhibiting production of TNF-α following LPS-stimulation of PBMC and human whole blood were ∼24 nM (6.55 ng/mL) and ∼25 nM (6.83 ng/mL), respectively. *In vitro* studies suggest a pharmacological activity profile for 3-(4-amino-1-oxo-1,3-dihydro -isoindol-2-yl)-piperidine-2,6-dione that is similar to, but at least 200 times more potent than, thalidomide. *In vitro* studies have also demonstrated that concentrations of 4-(amino)-2-(2,6-dioxo(3-piperidyl))-isoindoline-1,3-dione of 2.73 to 27.3 ng/mL (0.01 to 0.1 µM) achieved 50% inhibition of the proliferation of MM.IS and Hs Sultan cells.

The IC₅₀'s of 3-(4-amino-1-oxo-1,3-dihydro-isoindol-2-yl)-piperidine-2,6-dione for inhibiting production of TNF-α following LPS-stimulation of PBMC and human whole blood were ∼100 nM (25.9 ng/mL) and ∼480 nM (103.6 ng/mL), respectively. Thalidomide, in contrast, had an IC₅₀ of ∼194 µM (50.2 µg/mL) for inhibiting production of TNF-α following LPS-stimulation of PBMC. *In vitro* studies suggest a pharmacological activity profile for 3-(4-amino-1-oxo-1,3-dihydro-isoindol-2-yl)-piperidine-2,6-dione that is similar to, but 50 to 2000 times more potent than, thalidomide. It has been shown that the compound is approximately 50-100 times more potent than thalidomide in stimulating the proliferation of T-cells following primary induction by T-cell receptor (TCR) activation. 3-(4-amino-1-oxo-I,3-dihydro-isoindol-2-yl)-piperidine-2,6-dione is also approximately 50 to 100 times more potent than thalidomide in augmenting the production of IL-2 and IFN-γ following TCR activation of PBMC (IL-2) or T-cells (IFN-γ). In addition, 3-(4-amino-1-oxo-1,3-dihydro-isoindol-2-yl)-piperidine-2,6-dione exhibited dose-dependent inhibition of LPS-stimulated production of the pro-inflammatory cytokines TNF-α IL-1β, and IL-6 by PBMC while it increased production of the anti-inflammatory cytokine IL-10.

### INHIBITION OF MM CELL PROLIFERATION

The ability of 3-(4-amino-1-oxo-1,3-di-hydro-isoindol-2-yl)-piperidine-2,6-dione (Revimid^{™}) and thalidomide for comparison to effect the proliferation of MM cell lines has been investigated in an *in vitro* study. Uptake [³H]-thymidine by different MM cell lines (MM. IS, Hs Sultan, U266 and RPMI-8226) was measured as an indicator of cell proliferation. Cells were incubated in the presence of compounds for 48 hours; [³H]-thymidine was included for the last 8 hours of the incubation period. Addition of 3-(4-amino-1-oxo-1,3-dihydro-isoindol-2-yl)-piperidine-2,6-dione to MM.1S and Hs Sultan cells resulted in 50% inhibition of cell proliferation at concentrations of 0.4 µm and 1 µm, respectively. In contrast, addition of thalidomide at concentrations up to 100 µm resulted in only 15% and 20% inhibition of cell proliferation in MM.1S and Hs Sultan cells, respectively. These data are summarized in Figure 1.

### TOXICOLOGY STUDIES

The effects of 3-(4-amino-1-oxo-1,3-dihydro-isoindol-2-yl)-piperidine-2,6-dione (Revimid^{™}) on cardiovascular and respiratory function are investigated in anesthetized dogs. Two groups of Beagle dogs (2/sex/group) are used. One group receives three doses of vehicle only and the other receives three ascending doses of 3-(4-amino-1-oxo-1,3 -dihydro-isoindol-2-yl)-piperidine-2,6-dione (2,10, and 20 mg/kg). In all cases, doses of 3-(4-amino-1-oxo-1,3-dihydro-isoindol-2-yl)-piperidine-2,6-dione or vehicle are successively administered via infusion through the jugular vein separated by intervals of at least 30 minutes.

The cardiovascular and respiratory changes induced by 3-(4-amino-1-oxo-1,3 -dihydro-isoindol-2-yl)-piperidine-2,6-dione are minimal at all doses when compared to the vehicle control group. The only statistically significant difference between the vehicle and treatment groups is a small increase in arterial blood pressure (from 94 mmHg to 101 mmHg) following administration of the low dose of 3-(4-amino-1-oxo-1,3 -dihydro -isoindol-2-yl)-piperidine-2,6-dione. This effect lasts approximately 15 minutes and is not seen at higher doses. Deviations in femoral blood flow, respiratory parameters, and Qtc interval are common to both the control and treated groups and are not considered treatment-related.

### CYCLING THERAPY IN PATIENTS

In a specific embodiment, an immunomodulatory compound is cyclically administered to patients with cancer. Cycling therapy involves the administration of a first agent for a period of time, followed by a rest for a period of time and repeating this sequential administration. Cycling therapy can reduce the development of resistance to one or more of the therapies, avoid or reduce the side effects of one of the therapies, and/or improves the efficacy of the treatment.

In the embodiment, prophylactic or therapeutic agents are administered in a cycle of about 4 to 6 weeks, about once or twice every day. One cycle can comprise the administration of a therapeutic on prophylactic agent for three to four weeks and at least a week or two weeks of rest. The number of cycles administered is from about one to about 24 cycles, more typically from about two to about 16 cycles, and more typically from about four to about eight cycles.

For example, in a cycle of four weeks, on day 1, the administration of 25 mg/d of 3-(4-amino-1-oxo-1,3-dihydro-isoindol-2-yl)-piperidine-2,6-dione is started. On day 22, the administration of the compound is stopped for a week of rest. On day 29, the administration of 25 mg/d 3-(4-amino-1-oxo-1,3-dihydro-isoindol-2-yl)-piperidin-2,6-dione is begun.

### CLINICAL STUDIES IN PATIENTS

### TREATMENT OF RELAPSED MULTIPLE MYELOMA

4-(amino)-2-(2,6-dioxo(3-piperidyl))-isoindoline-1,3-dione (Actimid^{™}) was administered to patients with relapsed/refractory multiple myeloma. The study was conducted in compliance with Good Clinical Practices. Patients were at least 18 years old, had been diagnosed with multiple myeloma (with paraprotein in serum and/or urine), and were considered refractory to treatment after at least two cycles of treatment, or have relapsed after two cycles of treatment.

Patients who have progressive disease, according to the Southwest Oncology Group (SWOG) criteria, on their prior regimen are considered treatment refractory. Relapse following remission is defined as >25% increase in M component from baseline levels; reappearance of the M paraprotein that had previously disappeared; or a definite increase in the size and number of lytic bone lesions recognized on radiographs. Patients may have had prior therapy with thalidomide, provided they were able to tolerate the treatment. A Zubrod performance status of 0 to 2 is required for all patients.

4-(amino)-2-(2,6-dioxo(3-piperidyl))-isoindoline-1,3-dione is administered to patients at doses of 1, 2, 5, or 10 mg/day for up to four weeks; at each dose level, three patients are initially enrolled. Dosing occurs at approximately the same time each morning; all doses are administered in the fasted state (no eating for at least two hours prior to dosing and two hours after dosing). 4-(amino)-2-(2,6-dioxo(3-piperidyl))-isoindoline-1,3-dione doses are administered in an ascending fashion such that patients in the first cohort receive the lowest dose of 4-(amino)-2-(2,6-dioxo(3-piperidyl))-isoindoline-1,3-dione (1 mg/day) and escalation to the next higher dose level occurs only following the establishment of safety and tolerability at the current dose. If one out of three patients at any dose level experience dose limiting toxicity (DLT), three additional patients are enrolled at that dose. If none of the three additional patients experience DLT, escalation to the next dose level occurs; dose escalations continue in a similar fashion until the MTD is established or the maximum daily dose (10 mg/day) is attained. However, if one of the three additional patients enrolled experiences DLT, the MTD has been reached. If two or more of the three additional patients enrolled experience DLT, the MTD is judged to have been exceeded and three additional patients are enrolled at the preceding dose level to confirm the MTD. Once the MTD has been identified, four additional patients are enrolled at that dose level so that a total of 10 patients is treated at the MTD.

Blood sampling for analysis of pharmacokinetic parameters is performed on Days 1 and 28 according to the following sampling schedule: pre-dose, 0.25, 0.5, 0.75, 1,1.5, 2, 2.5, 3, 4, 6, 8, 10, 12,18, and 24 hours post-dose. An additional blood sample is collected at each weekly visit for the determination of 4-(mnino)-2-(2,6-dioxo(3-piperidyl))-isoindoline-1,3-dione levels. Total urine collections are also made with urine pooled according to the following time intervals post-dose: 0 to 4, 4 to 8, 8 to 12, and 12 to 24 hours. Safety assessments are made by monitoring adverse events, vital signs, ECGs, clinical laboratory evaluations (blood chemistry, hematology, lymphocyte phenotyping, and urinalysis), and physical examination at specific times during the study.

Results of interim pharmacokinetic analyses obtained following single- and multiple-dose administration of 4-(amino)-2-(2,6-dioxo(3-piperidyl))-isoindoline-1,3-dione to multiple myeloma patients are presented below in Tables 1 and 2. These data show that 4-(amino)-2-(2,6-dioxo(3-piperidyl))-isoindoline-1,3-dione was steadily absorbed at all dose levels in relapsed multiple myeloma patients. Maximum plasma concentrations occurred at a median Tₘₐₓ of between 2.5 and 2.8 hours post-dose at Day 1 and between 3 and 4 hours post-dose at Week 4. At all doses, plasma concentrations declined in a monophasic manner after reaching C_{max.} The start of the elimination phase occurred between 3 and 10 hours post-dose at Day 1 and Week 4, respectively.

These data also showed that after 4 weeks of dosing, 4-(amino)-2-(2,6-dioxo(3-piperidyl))-isoindoline-1,3-dione accumulated to a small extent (mean accumulation ratios ∼1.02 to 1.52 and ∼0.94 to 1.62 for Cₘₐₓ and AUC_{(0.7)}, respectively). There was almost a dose proportional increase in AUC₍₀₋₇₎ and Cₘₐₓ values with increasing dose. A five-fold higher dose of 4-(amino)-2-(2,6-dioxo(3-piperidyl))-isoindoline-1,3-dione produced a 3.2- and 2.2-fold increase in Cₘₐₓ at Day 1 and Week 4, respectively. Similarly, a 5-fold increase in dose resulted in a 3.6- and 2.3-fold increase in AUC₍₀₋ᵣ₎, at Day 1 and Week 4, respectively.

**Table 1**

| Pharmacokinetic parameters of Actimid^{™} in relapsed multiple myeloma patients | | | | |
|---|---|---|---|---|
| **Parameter** | | **1 mg** **(N=6)** | **2 mg** **(N=2)** | **5 mg** **(N=3)** |
| **Day 1** | | | | |
| Cₘₐₓ | ng/mL | 15.03 (4.04) | 24.4* (12.1) | 48.56 (14.03) |
| tₘₐₓ | h | 3.3 (2.6) | 2.7* (0.3) | 2.3 (0.3) |
| AUC_{(0-∞)} | ng.h/mL | 152.90 (36.62) | 279.18 (51.10) | 593.10 (335.23) |
| AUC_{(0-τ)} | | 134.21 (27.14) | 249.57 (29.26) | 520.94 (267.32) |
| t½ | h | 7.3 (3.4) | 6.3 (1.4) | 6.5 (2.2) |
| CL/F | mL/min | 114.75 (29.20) | 121.43 (22.22) | 182.31 (117.06) |
| Vz/f | L | 69.55 (44.97) | 65.31 (2.80) | 87.24 (22.61) |

| | | | | |
|---|---|---|---|---|
| t = 24 hours N/A = not available | | | | |

**Table 2**

| Pharmacokinetic parameters of Actimid^{™} following multiple oral doses(1, 2, and 5 mg/day) in relapsed multiple myeloma patients | | | | |
|---|---|---|---|---|
| **Parameter** | | **1 mg** **(N=5)** | **2 mg** **(N=2)** | **5 mg** **(N=3)** |
| Week 4 | | | | |
| Cₘₐₓ | ng/mL | 23.20 (7.48) | 30.05* (15.64) | 58.07 (38.08) |
| tₘₐₓ | h | 3.6 (1.5) | 2.8* (0.3) | 5.0 (2.6) |
| AUC_{(0-∞)} | ng.h/mL | N/A | N/A | N/A |
| AUC_{(0-τ)} | | 239.31 (122.59) | 269.36 (186.34) | 597.24 (354.23) |
| t½ | h | 6.2* (0.6) | 7.7 (2.8) | 7.8 (4.0) |
| CL/F | mL/min | 87.85 (48.48) | 162.68 (112.54) | 207.50 (175.41) |
| Vz/f | L | 41.35* (8.84) | 95.04 (35.39) | 103.95 (27.25) |

| | | | | |
|---|---|---|---|---|
| τ = 24 hours N/A = not available * N = 3 patients | | | | |

### TREATMENT OF RELAPSED MULTIPLE MYELOMA

Two Phase 1 clinical studies of 3-(4-amino-1-oxo-1,3-dihydro-isoindol-2-yl) -piperidine-2,6-dione (Revimid^{™}) have been conducted to identify the maximum tolerated dose (MTD) in patients with refractory or relapsed multiple myeloma. These studies have also characterized the safety profile of 3-(4-amino-1-oxo-1,3-dihydro-isoindol-2-yl) -piperidine-2,6-dione when ascending doses of 3-(4-amino-1-oxo-1,3-dihydro-isoindol -2-yl)-piperidine-2,6-dione were given orally for up to 4 weeks. Patients started 3-(4-amino-1-oxo-1,3-dihydro-isoindol-2-yl) -piperidine -2,6-dione treatment at 5 mg/day with subsequent escalation to 10, 25, and 50 mg/day. Patients were enrolled for 28 days at their assigned dose, with the option of extended treatment for those who did not exhibit disease progression or experience dose limiting toxicity (DLT). Patients were evaluated for adverse events at each visit and the severity of these events was graded according to the National Cancer Institute (NCI) Common Toxicity Criteria. Patients were discontinued if they experienced DLT (Grade 3 or greater non-hematological, or Grade 4 hematological toxicity).

In this study, 27 patients were enrolled. All patients had relapsed multiple myeloma and 18 (72%) were refractory to salvage therapy. Among these patients, 15 had undergone prior autologous stem cell transplantation and 16 patients had received prior thalidomide treatment. The median number of prior regimens was 3 (range 2 to 6).

Blood and urine samples were collected for analysis of pharmacokinetic parameters on Days 1 and 28. Blood samples were collected according to the following sampling schedule: pre-dose, 0.25, 0.5, 0.75, 1, 1.5, 2, 2.5, 3, 4, 6, 8, 10, 12, 18, and 24 hours post-dose. In addition, a blood sample was collected at each weekly clinic visit for 3-(4-amino-1-oxo-1,3-dihydro-isoindol-2-yl)-piperidine-2,6-dione determination. Total urine was collected and pooled according to the following time intervals post-dose: 0 to 4,4 to 8, 8 to 12, and 12 to 24 hours. Response to treatment was assessed by M-protein quantification (by immunoelectrophoresis) from serum and a 24-hour urine collection, with creatinine clearance and 24-hour protein calculations undertaken at screening, baseline, Weeks 2 and 4, and monthly thereafter (or upon early termination). Bone marrow aspirations and/or tissue biopsy are also performed at Months 3, 6 and 12 if a patient's paraprotein serum concentration or 24-hour urine protein excretion declined to the next lower level, based on best response criteria. Preliminary results for the 28-day treatment period are summarized below.

Preliminary pharmacokinetic analyses based on these two studies indicated that AUC and Cₘₐₓ values increase proportionally with dose following single and multiple doses in multiple myeloma patients (as was seen in healthy volunteers). Further, there was no evidence of accumulation with multiple dosing as single dose AUC_{(o-∞)} was comparable to multiple dose AUC_{0-τ} following the same dose of 3-(4-amino-1-oxo-1,3-dihydro -isoindol-2-yl)-piperidine-2,6-dione. Similar to healthy volunteer studies, double peaks were observed. Exposure in multiple myeloma patients appeared to be slightly higher based on Cₘₐₓ and AUC values as compared to healthy male volunteers while clearance in multiple myeloma patients was lower than it was in healthy volunteers, consistent with their poorer renal function (both as a consequence of their age and their disease). Finally, 3-(4-amino-1-oxo-1,3-dihydro-isoindol-2-yl)-piperidine-2,6-dione half-live in patients was shorter than in healthy volunteers (mean 8 hours, ranging up to 17 hours).

In this study, the first cohort of 3 patients was treated for 28 days at 5 mg/day without any dose limiting toxicity (DLT). The second cohort of 3 patients subsequently commenced therapy at 10 mg/day. Patients in the second 10 mg/day of 3-(4-amino-1-oxo-1,3-dihydro-isoindol-2-yl)-piperidine-2,6-dione cohort tolerated treatment well.

### TREATMENT OF SOLID TUMORS

Study with 3-(4-amino-1-oxo-1,3-dihydro-isoindol-2-yl)-piperidine-2,6-dione (Revimid^{™}) was conducted in patients with varying types of solid tumors, including malignant melanoma (13), carcinoma of the pancreas (2), carcinoid-unknown primary (1), renal carcinoma (1), breast carcinoma (1) and NSCLC (2). Patients received 5 mg/day 3-(4-amino-1-oxo-1,3-dihydro-isoindol-2-yl)-piperidine-2,6-dione for seven days and are subsequently escalated every seven days to 10 mg/day, 25 mg/day, and 50 mg/day for a total of 4 weeks of treatment. Patients who, experienced clinical benefit were permitted to continue on treatment as Named Patients.

The study initially enrolled 20 patients and was subsequently amended to enroll 16 additional patients (adrenal carcinoma, NSCLC, malignant mesothelioma, breast cancer, malignant melanoma (8), renal cell cancer (4)) at a higher dose. The 16 additional patients were given weekly escalating doses of 25 mg/day, 50 mg/day, 75 mg/day, 100 mg/day, 125 mg/day, and 150 mg/day over a 6-week period with continuing treatment for an additional six weeks.

The study of Phase 1 study was designed to determine a maximum tolerated dose (MTD) of 3-(4-amino-1-oxo-1,3-dihydro-isoindol-2-yl)-piperidine-2,6-dione in patients with refractory solid tumors and/or lymphoma, as well as to characterize the pharmacokinetic and side effect profiles of 3-(4-amino-1-oxo-1,3-dihydro-isoindol-2-yl) -piperidine-2,6-dione in this patient population. The study design dictates that at least 3 patients must be enrolled at a dose level and have completed 28 days of treatment prior to enrollment of patients at the next higher dose level. Patients in the first cohort began dosing at 5 mg/day of 3-(4-amino-1-oxo-1,3-dihydro-isoindol-2-yl)-piperidine- 2,6-dione. Patients will be escalated to 10, 20, 25, and 30 mg/day provided there is no toxicity.

In this study, the MTD is defined as the highest dose level in which fewer than two of six patients treated did not experience Grade 3 or greater non-hematological toxicity or Grade 4 or greater hematological toxicity. If, at any given dose level in either study, one out of three patients experiences toxicity, three additional patients must be treated at that particular dose. If, however, two out of six patients experience DLT, the MTD is judged to have been exceeded. No further dose escalations are to occur and additional patients are to be enrolled at the previous dose level. The dose of 3-(4-amino-1-oxo-1,3-dihydroisoindol -2-yl)-piperidine-2,6-dione administered is escalated until the MTD is achieved or the maximum daily dose of is reached.

No DLTs were reported in the initial group of 20 patients enrolled in the study. Thirteen of the original 20 trial patients, along with 2 non-trial patients, continued on treatment as named patients at doses up to 150 mg/day.

### TREATMENT OF GLIOMAS

This study was performed to find toxicity in patients with recurrent, high-grade gliomas. The study is designed such that patients are given increasingly higher doses of 3-(4-amino-1-oxo-1,3-dihydro-isoindol-2-yl)-piperidine-2,6-dione until a maximum tolerated dose (MTD) is established. The study also seeks to obtain preliminary toxicity information and pharmacokinetic data on 3-(4-amino-1-oxo-1,3-dihydro-isoindol-2-yl) -piperidine-2,6-dione, as well as to develop exploratory data concerning surrogate end points of angiogenic activity *in vivo* using functional neuro-imaging studies, and *in vitro* assays of serum angiogenic peptides.

Patients enrolled in the first cohort receive 2.5 mg/m²/day for a 4-week cycle. During each 4-week cycle of therapy, 3-(4-amino-1-oxo-1,3-dihydro-isoindol-2-yl) -piperidine-2,6-dione administered once daily for 3 weeks followed by a week of rest. Patients who complete a treatment cycle may receive another cycle of 3-(4-amino-1 -oxo-1,3-dihydro-isoindol-2-yl)-piperidine-2,6-dione treatment if two criteria are met. First, the patient must have stable disease or have experienced a partial response or complete response, or the patient is benefiting from the therapy with 3-(4-amino-1-oxo-1,3-dihydro -isoindol-2-yl)-piperidine-2,6-dione as evidenced by a decrease in tumor-related symptoms such as neurological deficits. Second, the patient must have recovered from toxicity related to 3-(4-amino-1-oxo-1,3-dihydro-isoindol-2-yl) -piperidine-2,6-dione which occurred in the prior cycle by Day 42 or sooner (28-day cycle plus limit of 2 weeks to recover) as evidenced by a return to Grade ≤ 1 toxicity level. Patients who experience DLT in the previous cycle should have their dose modified. DLT is defined as an non-hematological event Grade ≥ 3 toxicity or hematological event of Grade 4 toxicity thought to be related to the study medication. Patients who experience DLT in the first cycle and have no response to therapy are removed from the study.

3-(4-amino-1-oxo-1,3-dihydro-isoindol-2-yl) -piperidine-2,6-dione doses are subsequently escalated to 5, 8, 11, 15, and 20 mg/m²/day to a maximum total daily dose of 40 mg. Patients continue to receive 3-(4-amino-1-oxo-1,3-dihydro-isoindol-2-yl)-piperidine -2,6-dione on a 4-week cycle per dose level until one of the off-study criteria are met.

Three patients are enrolled in each cohort. If at least one DLT occurs, three additional patients are added to the cohort at that particular dose level. If two DLTs occur, the MTD, defined as the dose at which fewer than one-third of patients at each dose level experiences DLT has been exceeded and four more patients are treated at the previous dose.

Patients who experience DLT during the first 4-week cycle are removed from the study, except if they have a response to therapy. For patients who have completed their first 4-week cycle of without DLT, but who subsequently experience Grade 3 or 4 hematological and/or nonhematological toxicity, treatment is suspended for a minimum of a week. If the toxicity resolves to < Grade 2 within three weeks, the patient is treated at two dose levels lower than the dose that caused the toxicity (or a 50% reduction if the patient was treated at the first or second dose level). Patients in whom Grade 3 or 4 toxicity does not resolve to < Grade 1 within three weeks, or those who have another Grade 3 toxicity at the reduced dose are removed from the study.

Pharmacokinetic sampling is performed prior the first dose of 3-(4-amino-1-oxo-1,3 -dihydro-isoindol-2-yl)-piperidine-2,6-dione (Day 1) and 0.5, 1, 2, 4, 6, 8, 24, and 48 hours thereafter. Sampling is also conducted pre-dose on Days 7 and 21 and 0.5, 1, 2, 4, 6, 8, and 24 post-dose on Day 21 to evaluate steady-state 3-(4-amino-1-oxo-1,3-dihydro-isoindol -2-yl)-piperidine-2,6-dione levels.

### TREATMENT OF METASTATIC MELANOMA

Patients with metastatic melanoma were started on 3-(4-amino-1-oxo-1,3-dihydro -isoindol-2-yl)-piperidine-2,6-dione (Revmid^{™}) at 5 mg/day for seven days. The dose was then increased every seven days to 10 mg/day, 25 mg/day, and 50 mg/day, respectively, for a total of four weeks on therapy. Five of the 13 melanoma patients who were treated under this regimen either showed disease stabilization or a partial response in the first four weeks of treatment. Tumor response was seen in cutaneous and subcutaneous lesions (five patients), lymph nodes (two patients), and liver (one patient). The duration of response was approximately six months. The result suggests that the compound appears is a promising new anti-cancer agent and has both antiangiogenic and immunomodulatory properties.

### TREATMENT OF RELAPSED OR REFRACTORY MULTIPLE MYELOMA

Patients with relapsed and refractory Dune-Salmon stage III multiple myeloma, who have either failed at least three previous regimens or presented with poor performance status, neutropenia or thrombocytopenia, are treated with up to four cycles of combination of melphalan (50 mg intravenously), an immunomodulatory compound (about 1 to 150 mg orally daily), and dexamethasone (40 mg/day orally on days 1 to 4) every four to six weeks. Maintenance treatment consisting of daily an immunomodulatory compound and monthly dexamethasone are continued until the disease progression. The therapy using an immunomodulatory compound in combination with melphalan and dexamethasone is highly active and generally tolerated in heavily pretreated multiple myeloma patients whose prognosis is otherwise poor,

The embodiments described above are intended to be merely exemplary, and those skilled in the art will recognize, or will be able to ascertain using no more than mutine experimentation, numerous equivalents of specific compounds, materials, and procedures.

## Claims

1. Use of a therapeutically effective amount of a compound of the formula 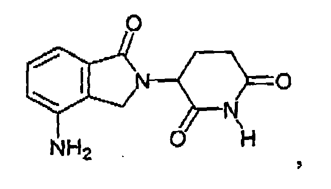 or a pharmaceutically acceptable salt, solvate or hydrate thereof, and a therapeutically effective amount of a second active ingredient, in the preparation of a pharmaceutical composition for treating multiple myeloma,
(i) wherein the second active ingredient is dexamethasone;
(ii) wherein the multiple myeloma is relapsed or refractory myeloma, and wherein the second active ingredient is a combination of doxorubicin and dexamethasone; or
(iii) wherein the multiple myeloma is relapsed or refractory myeloma, and wherein the second active ingredient is a combination of doxorubicin, vincristin and dexamethasone.

2. Use of claim 1, wherein the multiple myeloma is relapsed or refractory myeloma and wherein said pharmaceutical composition is for administration of the compound in an amount of 15 mg/day twice a day or 30 mg/day four times a day in combination with dexamethasone.

3. Combination of a therapeutically effective amount of a compound of the formula 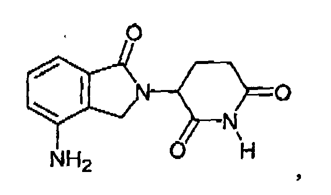 a pharmaceutically acceptable salt, solvate or hydrate thereof, and a therapeutically effective amount of a second active ingredient for use in treating multiple myeloma,
(i) wherein the second active ingredient is dexamethasone;
(ii) wherein the multiple myeloma is relapsed or refractory myeloma, and wherein the second active ingredient is a combination of doxorubicin and dexamethasone; or
(iii) wherein the multiple myeloma is relapsed or refractory myeloma, and wherein the second active ingredient is a combination of doxorubicin, vincristin and dexamethasone.

4. Combination of claim 3, wherein the multiple myeloma is relapsed or refractory myeloma and wherein the combination is for administration of the compound in an amount of 15 mg/day twice a day or 30 mg/day four times a day in combination with dexamethasone.

5. Pharmaceutical composition comprising a therapeutically effective amount of a compound of the formula 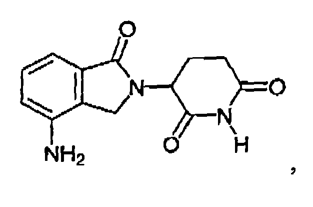 a pharmaceutically acceptable salt, solvate or hydrate thereof, and a therapeutically effective amount of a second active ingredient for use in treating multiple myeloma,
(i) wherein the second active ingredient is dexamethasone;
(ii) wherein the multiple myeloma is relapsed or refractory myeloma, and wherein the second active ingredient is a combination of doxorubicin and dexamethasone; or
(iii) wherein the multiple myeloma is relapsed or refractory myeloma, and wherein the second active ingredient is a combination of doxorubicin, vincristin and dexamethasone.

## Patentansprüche

1. Verwendung einer therapeutisch wirksamen Menge einer Verbindung der Formel 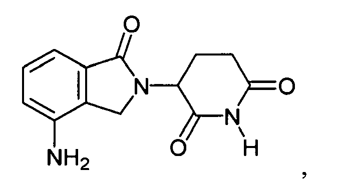 oder eines pharmazeutisch annehmbaren Salzes, Solvats oder Hydrats davon, und einer therapeutisch wirksamen Menge eines zweiten Wirkstoffs zur Herstellung einer pharmazeutischen Zusammensetzung zur Behandlung von multiplem Myelom,
(i) wobei der zweite Wirkstoff Dexamethason ist;
(ii) wobei das multiple Myelom rezidives oder refraktäres Myelom ist, und wobei der zweite Wirkstoff eine Kombination aus Doxorubicin und Dexamethason ist; oder
(iii) wobei das multiple Myelom rezidives oder refraktäres Myelom ist, und wobei der zweite Wirkstoff eine Kombination aus Doxorubicin, Vincristin und Dexamethason ist.

2. Verwendung nach Anspruch 1, wobei das multiple Myelom rezidives oder refraktäres Myelom ist und wobei die pharmazeutische Zusammensetzung zur Verabreichung der Verbindung in einer Menge von 15 mg/Tag zwei Mal täglich oder 30 mg/Tag vier Mal täglich in Kombination mit Dexamethason dient.

3. Kombination einer therapeutisch wirksamen Menge einer Verbindung der Formel 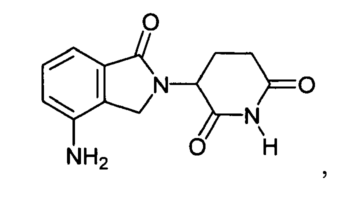 eines pharmazeutisch annehmbaren Salzes, Solvats oder Hydrats davon, und einer therapeutisch wirksamen Menge eines zweiten Wirkstoffs zur Verwendung zur Behandlung von multiplem Myelom,
(i) wobei der zweite Wirkstoff Dexamethason ist;
(ii) wobei das multiple Myelom rezidives oder refraktäres Myelom ist, und wobei der zweite Wirkstoff eine Kombination aus Doxorubicin und Dexamethason ist; oder
(iii) wobei das multiple Myelom rezidives oder refraktäres Myelom ist, und wobei der zweite Wirkstoff eine Kombination aus Doxorubicin, Vincristin und Dexamethason ist.

4. Kombination nach Anspruch 3, wobei das multiple Myelom rezidives oder refraktäres Myelom ist und wobei die Kombination zur Verabreichung der Verbindung in einer Menge von 15 mg/Tag zwei Mal täglich oder 30 mg/Tag vier Mal täglich in Kombination mit Dexamethason dient.

5. Pharmazeutische Zusammensetzung umfassend eine therapeutisch wirksame Menge einer Verbindung der Formel 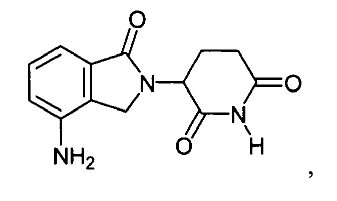 eines pharmazeutisch annehmbaren Salzes, Solvats oder Hydrats davon, und eine therapeutisch wirksame Menge eines zweiten Wirkstoffs zur Verwendung zur Behandlung von multiplem Myelom,
(i) wobei der zweite Wirkstoff Dexamethason ist;
(ii) wobei das multiple Myelom rezidives oder refraktäres Myelom ist, und wobei der zweite Wirkstoff eine Kombination aus Doxorubicin und Dexamethason ist; oder
(iii) wobei das multiple Myelom rezidives oder refraktäres Myelom ist, und wobei der zweite Wirkstoff eine Kombination aus Doxorubicin, Vincristin und Dexamethason ist.

## Revendications

1. Utilisation d'une quantité thérapeutiquement efficace d'un composé selon la formule 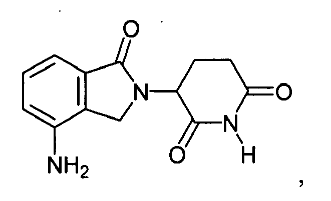 ou d'un sel, solvate ou hydrate pharmaceutiquement acceptables de celui-ci, et d'une quantité pharmaceutiquement efficace d'un deuxième agent actif dans la préparation d'une composition pharmaceutique pour le traitement du myélome multiple,
(i) où le deuxième agent actif est dexaméthasone;
(ii) où le myélome multiple est le myélome récidiviste ou réfractaire et où le deuxième agent actif est une combinaison de doxorubicine et dexaméthasone; ou
(iii) où le myélome multiple est le myélome récidiviste ou réfractaire et où le deuxième agent actif est une combinaison de doxorubicine, vincristine et dexaméthasone.

2. Utilisation selon la revendication 1, où le myélome multiple est le myélome récidiviste ou réfractaire et où la composition pharmaceutique est destinée à l'administration du composé dans une quantité de 15 mg/jour deux fois par jour ou 30 mg/jour quatre fois par jour en combinaison avec dexaméthasone.

3. Combinaison d'une quantité thérapeutiquement efficace d'un composé selon la formule 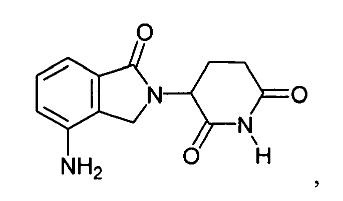 d'un sel, solvate ou hydrate pharmaceutiquement acceptables de celui-ci, et d'une quantité pharmaceutiquement efficace d'un deuxième agent actif pour utilisation dans le traitement du myélome multiple,
(i) où le deuxième agent actif est dexaméthasone;
(ii) où le myélome multiple est le myélome récidiviste ou réfractaire et où le deuxième agent actif est une combinaison de doxorubicine et dexaméthasone; ou
(iii) où le myélome multiple est le myélome récidiviste ou réfractaire et où le deuxième agent actif est une combinaison de doxorubicine, vincristine et dexaméthasone.

4. Combinaison selon la revendication 3, où le myélome multiple est le myélome récidiviste ou réfractaire et où la combinaison est destinée à l'administration du composé dans une quantité de 15 mg/jour deux fois par jour ou 30 mg/jour quatre fois par jour en combinaison avec dexaméthasone.

5. Composition pharmaceutique comprenant une quantité thérapeutiquement efficace d'un composé selon la formule 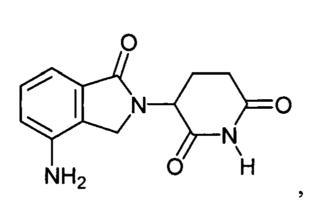 d'un sel, solvate ou hydrate pharmaceutiquement acceptables de celui-ci, et une quantité pharmaceutiquement efficace d'un deuxième agent actif pour utilisation dans le traitement du myélome multiple,
(i) où le deuxième agent actif est dexaméthasone ;
(ii) où le myélome multiple est le myélome récidiviste ou réfractaire et où le deuxième agent actif est une combinaison de doxorubicine et dexaméthasone ; ou
(iii) où le myélome multiple est le myélome récidiviste ou réfractaire et où le deuxième agent actif est une combinaison de doxorubicine, vincristine et dexaméthasone.
